# EUROPEAN PATENT APPLICATION

(11) **EP 1 285 634 A1**
(43) Date of publication of application: **26.02.2003**
(21) Application number: 02255474.5
(22) Date of filing: 05.08.2002
(51) Int. Cl.: A61B 19/00

(54) **Robotically controlled surgical instrument with visual force-feedback**

(30) Priority: 21.08.2001 US 935555
(71) Applicant: COMPUTER MOTION, INC., Goleta, CA 93117 (US)
(72) Inventor: Wang, Yulun, Goleta, California 93117 (US); Holz, Brian, Santa-Barbara, California 93111 (US); Miller, Brian, Santa-Barbara, California 93111 (US); Belinski, Steve, Santa-Barbara, California 93110 (US)
(74) Representative: Wombwell, Francis

(57) **Abstract**

A robotic medical system that provides a visual indication of the force exerted by an end effector of the system. The end effector is coupled to a robotic arm that is controlled by a handle. The end effector is inserted through an incision of a patient to perform a minimally invasive procedure. The surgeon can view the procedure on a screen that is coupled to an endoscope also inserted into the patient. The visual indicator may be a bar graph displayed on the screen. The bar graph has individual bar segments that are illuminated in accordance with the amplitude of the force exerted by the end effector. The visual force indicator provides a high resolution force feedback system for the instrument end effector.

## Description

### 1. FIELD OF THE INVENTION

The present invention relates a medical robotic system.

### 2. BACKGROUND INFORMATION

Blockage of a coronary artery may deprive the heart of blood and oxygen required to sustain life. The blockage may be removed with medication or by an angioplasty. For severe blockage a coronary artery bypass graft (CABG) is performed to bypass the blocked area of the artery. CAEG procedures are typically performed by splitting the sternum and pulling open the chest cavity to provide access to the heart. An incision is made in the artery adjacent to the blocked area. The internal mammary artery is then severed and attached to the artery at the point of incision. The internal mammary artery bypasses the blocked area of the artery to again provide a full flow of blood to the heart. Splitting the sternum and opening the chest cavity can create a tremendous trauma to the patient. Additionally, the cracked sternum prolongs the recovery period of the patient.

Computer Motion of Goleta, California provides a system under the trademark ZEUS that allows a surgeon to perform a minimally invasive CABG procedure. The procedure is performed with instruments that are inserted through small incisions in the patient's chest. The instruments are controlled by robotic arms. Movement of the robotic arms and actuation of the instrument end effectors are controlled by the surgeon through a pair of handles and a foot pedal that are coupled to an electronic controller. Alternatively, the surgeon can control the movement of an endoscope used to view the internal organs of the patient through voice commands.

The instrument end effectors may include scissors, graspers, or other devices that apply a force to the patient. The handles used to move the instruments are electronically coupled to the instrument end effectors. With an electronically coupled system there is no mechanical feedback on the amount of force that is being applied by the end effector. Feedback is desired to allow the surgeon to "feel" the force being applied to the patient. The Zeus system compensates for the lack of mechanical feedback by incorporating a force sensor that is coupled to the end effectors and actuators in the handles. The actuators in the handles exert a force on the surgeon which corresponds to the amount of force being applied by the end effector onto the patient.

The mechanical actuators and mechanical/electrical transducers may create slight errors in the force feedback system. The fidelity of the feedback system maybe such that the force exerted on the surgeon is different than the force applied by the end effector For example, the surgeon may feel like the instrument is applying more force than what is actually being applied by the end effector. This may create complications in the surgical procedure. Additionally, the feedback force exerted by the handles can cause fatigue in the surgeon's hands.

### SUMMARY OF THE INVENTION

A medical system that may include a visual indicator coupled to a force sensor of an end effector. The end effector may be coupled to a robotic arm that is coupled to a handle.

### BRIEF DESCRIPTION OF THE DRAWINGS

Figure 1 is a perspective view of a medical system;
Figure 2 is a side view of a surgical instrument;
Figure 3 is a front view of a monitor screen showing a visual force indicator;
Figure 4 is a top view of a pendent screen showing a graphical user interface;
Figure 5 is a top view of the pendent screen showing a different graphical user interface;
Figure 6 is a top view of the pendent screen showing a bar graph that provides a visual force indicator;
Figure 7 is a top view of a visual force indicator.

### DETAILED DESCRIPTION

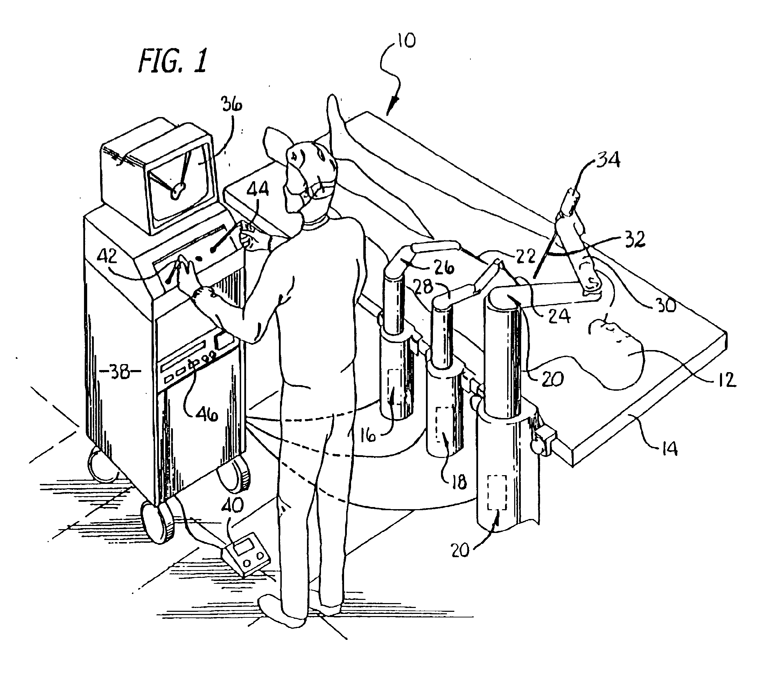

Referring to the drawings more particularly by reference numbers, Figure 1 shows a system 10 that can perform minimally invasive surgery. In one embodiment, the system 10 is used to perform a minimally invasive coronary artery bypass graft (MI-CABG) and other anastomostic procedures. Although a MI-CABG procedure is shown and described, it is to be understood that the system may be used for other surgical procedures. For example, the system can be used to suture any pair of vessels. The system 10 can be used to perform a procedure on a patient 12 that is typically lying on an operating table 14. Mounted to the operating table 14 is a first articulate arm 16, a second articulate arm 18 and a third articulate arm 20. The articulate arms 16, 18 and 20 are preferably mounted to the table 14 so that the arms are at a same reference plane as the patient. Although three articulate arms are shown and described, it is to be understood that the system may have any number of arms.

The first and second articulate arms 16 and 18 each have a surgical instrument 22 and 24, respectively, coupled to robotic arms 26 and 28, respectively. The third articulate arm 20 includes a robotic arm 30 that holds and moves an endoscope 32. The instruments 22 and 24, and endoscope 32 are inserted through incisions cut into the skin of the patient. The endoscope has a camera 34 that is coupled to a television monitor 36 which displays images of the internal organs of the patient. The first 16, second 18, and third 20 articulate arms are coupled to a controller 38 which can control the movement of the arms. The controller 38 is connected to an input device 40 such as a foot pedal that can be operated by a surgeon to move the location of the endoscope 28. The controller 38 contains electrical circuits, such as a processor, to control the robotic arms 26, 28 and 30. The surgeon can view a different portion of the patient by depressing a corresponding button(s) of the pedal 40. The controller 38 receives the input signal(s) from the foot pedal 40 and moves the robotic arm 30 and endoscope 32 in accordance with the input commands of the surgeon. The robotic arms 26, 28 and 30 may be devices that are sold by the assignee of the present invention, Computer Motion, Inc. of Goleta, California, under the trademark AESOP. The system is also described in U.S. Patent No. 5,657,429 issued-to Wang et al., which is hereby incorporated by reference. Although a foot pedal 40 is shown and described, it is to be understood that the system may have other input means such as a hand controller, or a speech recognition interface.

The instruments 22 and 24 of the first 16 and second 18 articulate arms, respectively, are controlled by a pair of master handles 42 and 44 that can be manipulated by the surgeon. The handles 42 and 44, and arms 16 and 18, have a master-slave relationship so that movement of the handles 42 and 44 produces a corresponding movement of the surgical instruments 22 and 24. The handles 42 and 44 may be mounted to a portable cabinet 46. The handles 42 and 46 are also coupled to the controller 38. The controller 38 receives input signals from the handles 42 and 44, computes a corresponding movement of the surgical instruments, and provides output signals to move the robotic arms 26 and 28 and instruments 22 and 24. The entire system may be a product marketed by Computer Motion under the trademark ZEUS. The operation of the system is also described in U.S. Patent No. 5,762,458 issued to Wang et al. and assigned to Computer Motion, which is hereby incorporated by reference.

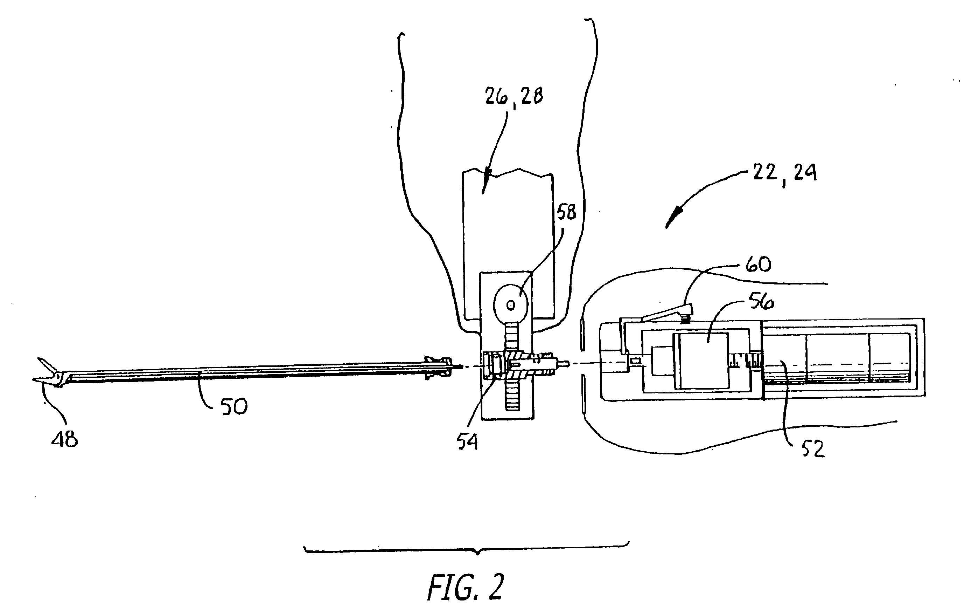

Figure 2 shows one of the surgical instruments 22 or 24. The instruments 22 or 24 includes an end effector 48 that is coupled to an actuator rod 50. The actuator rod 50 is coupled to a motor 52 by an adapter 54. The motor 52 actuates the end effector 48 by moving the actuator rod 50. The actuator rod 50 is coupled to a force sensor 56 that can sense the force being applied by the end effector 48. The force sensor 56 provides an analog output signal that is sent to the controller shown in Fig. 1.

The adapter 54 is coupled to a gear assembly 58 located at the end of a robotic arm 26 or 28. The gear assembly 58 can rotate the adapter 54 and end effector 48. The actuator rod 50 and end effector 48 may be coupled to the force sensor 56 and motor 52 by a spring biased lever 60. The instrument 22 or 24 may be the same or similar to an instrument described in the '458 patent.

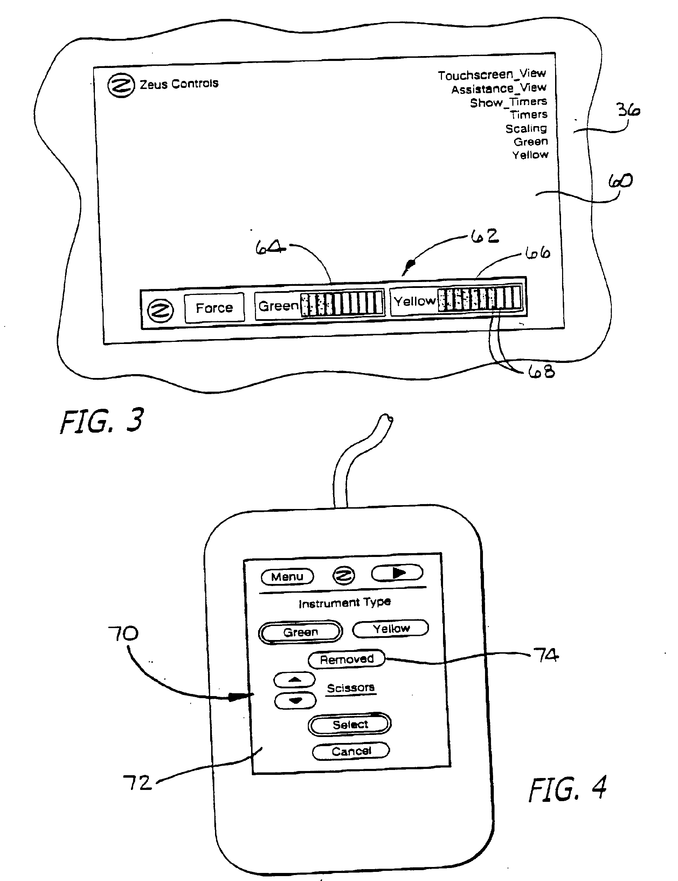

As shown in Figure 3, the monitor 36 has a screen 60 that displays a visual force indicator 62. The indicator 62 may be a graphic that is generated by the controller (38 in Fig. 1). The graphic may include a pair of bar graphs 64 and 66 that provide an indication of the amplitude of the force being exerted by instruments 22 and 24, respectively. The bar graphs 64 and 66 may each include separate bar segments 68 that are illuminated in a sequential manner. The number of illuminated bar segments 68 correspond to the force being exerted by the instrument. The controller 38 generates and varies the bar graphs 64 and 66 in response to the analog output signal provided by the force sensors of the instruments. For example, a greater force will increase the output signal. The processor will process the increased output signal to illuminate more bar segments 68. Likewise, the processor will illuminate less bar segments 68 in response to a lower output signal and instrument force. Although bar graphs 64 and 66 are shown and described, it is to be understood that the visual force indicator may be some other type of graphic, or video signal.

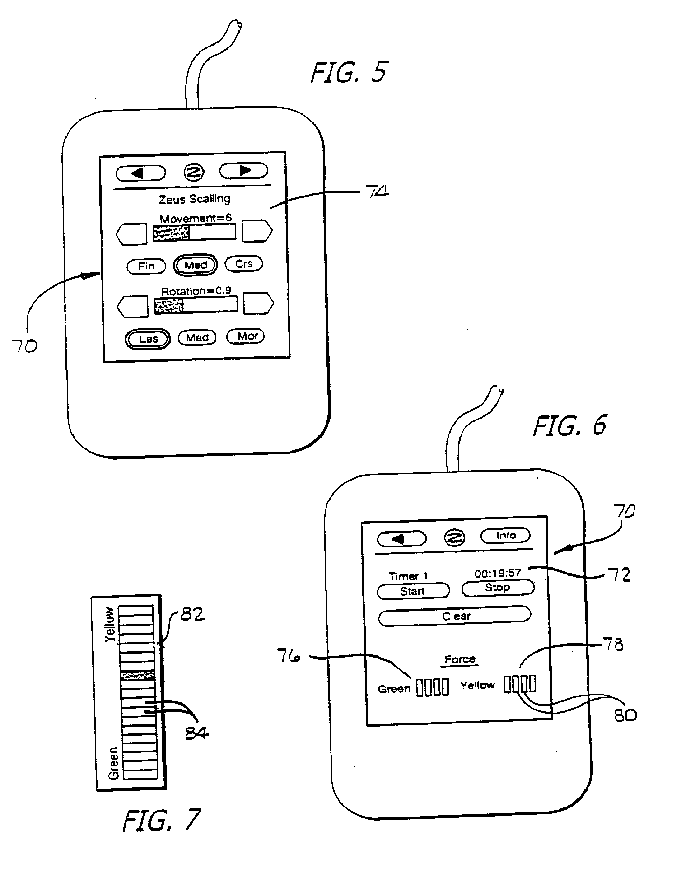

Figure 4 shows a hand pendent 70 that can be used to provide inputs to the controller. The pendent 70 can be the same device sold by Computer Motion under the trademark HERMES. The pendent 70 includes a screen 72 that displays a graphical user interface ("GUI") 74. The GUI 74 may allow the operator to select a function or input parameter by touching the screen 70. For example, as shown in Figure 5 the GUI 74 may allow the operator to vary the scale between the movement of the system handles and the corresponding linear and rotational movement of the end effector.

As shown in Figure 6, the pendent screen 72 may display a pair of bar graphs 76 and 78 that correspond to the force exerted by the instruments. The bar graphs 76 and 78 may each contain bar segments 80 that are individually illuminated in accordance with the instrument forces. An increase in force will illuminate more bar segments 80. Likewise, a lower force will decrease the number of illuminated bar segments 80.

Figure 7 shows another embodiment that includes a display 82 which contains a number of light emitting diodes ("LEDs") 84 that can be illuminated in accordance with the force exerted by a corresponding instrument. The display 82 may be a separate device mounted to any area of the system that is visually apparent to the operator. By wasy of example, the display 82 may be coupled to the handles of the system 10.

Providing a visual indicator that is generated by an electronic controller eliminates mechanical actuators in the handles and provides a force feedback with a greater resolution than mechanical feedback systems of the prior art. Additionally, the visual system does not create human fatigue found in prior mechanical feedback systems.

While certain exemplary embodiments have been described and shown in the accompanying drawings, it is to be understood that such embodiments are merely illustrative of and not restrictive on the broad invention, and that this invention not be limited to the specific constructions and arrangements shown and described, since various other modifications may occur to those ordinarily skilled in the art.

## Claims

1. A medical system, comprising:
a robotic arm;
an end effector coupled to said robotic arm;
a force sensor coupled to said end effector;
a handle coupled to said robotic arm; and,
a visual force indicator coupled to said force sensor.

2. The medical system of claim 1, further comprising a monitor, wherein said visual force indicator includes a graphic displayed on said monitor.

3. The medical system of claim 1, wherein said graphic varies in accordance with a change in a force sensed by said force sensor.

4. The medical system of claim 3, wherein the graphic includes a bar graph.

5. The medical system of claim 1, wherein said visual force indicator is coupled to said handle.

6. The medical system of claim 5, wherein said visual force indicator includes a plurality of light emitting diodes.

7. The medical system of claim 1, further comprising a pendent, wherein said visual force indicator includes a graphic displayed on said pendent.

8. The medical system of claim 7, wherein said graphic varies in accordance with a change in a force sensed by said force sensor.

9. The medical system of claim 8, wherein the graphic includes a bar graph.

10. A medical system, comprising:
a robotic arm;
an end effector coupled to said robotic arm;
force sensor means for sensing a force applied by said end effector;
a handle coupled to said robotic arm; and,
indicator means for providing an visual indication that correlates to the force sensed by said force sensor means.

11. The medical system of claim 10, further comprising a monitor, wherein said indicator means includes a graphic displayed on said monitor.

12. The medical system of claim 10, wherein said graphic varies in accordance with a change in the force sensed by said force sensor means.

13. The medical system of claim 12, wherein the graphic includes a bar graph.

14. The medical system of claim 10, wherein said indicator means is coupled to said handle.

15. The medical system of claim 14, wherein said indicator means includes a plurality of light emitting diodes.

16. The medical system of claim 10, further comprising a pendent, wherein said indicator means includes a graphic displayed on said pendent.

17. The medical system of claim 16, wherein said graphic varies in accordance with a change in a force sensed by said force sensor means.

18. The medical system of claim 17, wherein the graphic includes a bar graph.

19. A method for providing a visual indication of a force exerted by an end effector of a medical robotic system, comprising:
inserting an end effector through an incision in a patient;
actuating the end effector to exert a force on the patient;
sensing the force exerted on the patient; and, displaying a visual indication of the force.

20. The method of claim 19, wherein the visual indication is displayed on a monitor.

21. The method of claim 20, wherein the visual indication includes a bar graph.

22. The method of claim 19, wherein the visual indication is displayed on a pendent.

23. The method of claim 22, wherein the visual indication includes a bar graph.
